(19) Europäisches Patentamt
European Patent Office
Office européen des brevets

(11) **EP 4 246 146 A1**

(12) **EUROPEAN PATENT APPLICATION**

(43) Date of publication:
**20.09.2023 Bulletin 2023/38**

(21) Application number: **22382244.6**

(22) Date of filing: **14.03.2022**

(51) International Patent Classification (IPC):
*G01N 33/574* $^{(2006.01)}$

(52) Cooperative Patent Classification (CPC):
**G01N 33/57438; G01N 33/574; G01N 33/57411;
G01N 33/57423; G01N 33/57442; G01N 33/57446;
G01N 33/57449;** G01N 2570/00

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Fundació Institut Mar d'Investigacions Mèdiques
(IMIM)
08003 Barcelona (ES)**
• **Universitat de Girona
17003 Girona (ES)**
• **Fundacio Institut d'Investigació Biomèdica de
Girona Dr. Josep Trueta
17190 Salt (Girona) (ES)**
• **Institut Català D'Oncologia
08908 L'Hospitalet de Llobregat (ES)**

(72) Inventors:
• **POZO MENDOZA, Óscar Juan
08202 SABADELL (ES)**
• **GÓMEZ GÓMEZ, Àlex
08031 BARCELONA (ES)**

• **GIL GÓMEZ, Gabriel
08018 BARCELONA (ES)**
• **PERA ROMAN, Manuel Roman
08017 BARCELONA (ES)**
• **VISA TURMO, Laura
08025 BARCELONA (ES)**
• **ARRIOLA APERRIBAY, Edurne
08018 BARCELONA (ES)**
• **PERACAULA MIRÓ, Rosa
17003 GIRONA (ES)**
• **LLOP ESCORIHUELA, Esther
17003 GIRONA (ES)**
• **CASTRO GUTIÉRREZ, Ernesto
08290 BARCELONA (ES)**
• **GARCÍA VELASCO, Adelaida
17240 GIRONA (ES)**
• **FORT MARTORELL, Esther
17240 SALT (ES)**
• **PÉREZ DEL CAMPO, Dunia
17003 GIRONA (ES)**

(74) Representative: **ZBM Patents - Zea, Barlocci &
Markvardsen
Rambla Catalunya, 123
08008 Barcelona (ES)**

(54) **METHOD FOR DIAGNOSING CANCER**

(57)     The invention refers to an in vitro method for diagnosing cancer in a subject, said method comprising the steps of: (i) determining, in a sample obtained from the subject, the concentration of (a) 3-oxopropanoate, and at least one further metabolite selected from (b) a ketone body selected from beta-hydroxybutyrate, acetoacetate and combinations thereof, and (c) a medium chain fatty acid selected from octanoate, decanoate and combinations thereof, (ii) comparing the concentration of each of the metabolites determined in (i) with a corresponding reference value, and (iii) diagnosing the patient as having cancer when the concentration of at least one of the metabolites is increased as compared to the corresponding reference value.

EP 4 246 146 A1

**Description**

**Technical Field**

The application refers to the field of cancer diagnosis and, in particular, to biomarkers for diagnosing cancer.

**Background Art**

Cancer is one of the most devastating diseases worldwide. Pancreatic cancer, in particular, has become the third leading cause of cancer-related death in both sexes in developed countries (Siegel RL, et al, CA Cancer J Clin. 2022 Jan;72(1):7-33). Its incidence is increasing, with the projection to be the second-leading cause of cancer death by 2030 in the US (Rahib L, et al, Cancer Research. 2014 Jun;74(11):2913-2921).

The majority of the diagnosed pancreatic tumors (90%) are ductal adenocarcinomas (PDAC), which is one of the most lethal cancers, with a 5-year survival of less than 10% (Park W, et al,JAMA - Journal of the American Medical Association. 2021 Sep;326(9):851-862). The reasons for this dismal prognosis are its insidious onset (most patients present with nonspecific symptoms even in the presence of locally advanced, surgically unresectable disease or distant metastasis) and the modest efficacy of the adjuvant therapy. It has been estimated that earlier diagnosis of PDAC at a resectable stage could improve five-year survival up to almost 40% (Siegel RL, et al, CA Cancer J Clin. 2022 Jan;72(1):7-33; Kenner B, et al, Pancreas. 2021 Mar;50(3):251-279), emphasizing the importance of early diagnosis to reduce death rates. The only potentially curative treatment for PDAC is radical resection with tumour free margins although, because of the late diagnosis, less than 20% of the patients can benefit of this approach due to the presence of locally advanced disease or metastasis at the time of diagnosis (Gillen S, et al, PLoS Medicine. 2010 Apr;7(4):e1000267). Surveillance programs are recommended for groups at high-risk of developing PDAC: patients with germline mutations in DNA repair genes such BRCA1/2 or family history of pancreatic cancer (mostly PRSS1 mutations), patients with mucinous cysts (mucinous cystic neoplasms, MCN, and intraductal papillary mucinous neoplasms, IPMN), new onset-diabetes (patients diagnosed at age 50 and older) and patients with chronic pancreatitis. However, the screening of the general population for pancreatic cancer, as for other types of cancer, is not recommended due, among other factors, to the low sensitivity of the available tests for early stages.

Currently, the only clinically established biomarker for PDAC is Carbohydrate Antigen 19-9 (CA19-9). It is widely used in the monitoring of disease progression and the response to chemotherapy. However, it has a low positive predictive value due to the relatively low incidence of pancreatic cancer in asymptomatic subjects and lacks the specificity needed for early detection of the disease (Luo G, et al, Biochim Biophys Acta Rev Cancer. 2021 Apr;1875(2):188409). Similar problems are found for early diagnosis of other cancer types such as gastric, esophageal, lung or ovarian cancer.

Identification of biomarkers able to detect pancreatic cancer, such as PDAC, and other types of cancer at early stages using sensitive, specific, reliable, practical and cost-effective techniques is an urgent unmet medical need. Many studies have reported the use of tumor biopsy samples obtained during surgery or by ultrasound-guided fine needle aspiration or blood samples to identify genomic, proteomic, glycomic and metabolomics markers for cancer. However, none of these markers have been proved useful in the context of early diagnosis (surveillance biomarkers). On the other hand, tests described for early disease, such as a 29-protein marker signature (Mellby et al, J Clin Oncol. 2018 Oct 1;36(28):2887-2894), may have limited application to large populations because of their complexity.

There remains a need to provide predictive biomarker-based methods and tests for effectively diagnosing cancer which may be economically feasible for implementing in the clinical practice. Moreover, there is a need for these tools to be able to diagnose early-stage cancer.

**Summary of Invention**

The present inventors have surprisingly found that a single metabolic signature based on three metabolites may be used for diagnosing several types of cancer with good sensitivity and specificity.

Thus, a first aspect of the invention refers to an *in vitro* method for diagnosing cancer in a subject, said method comprising the steps of:

(i) determining, in a sample obtained from the subject, the concentration of (a) 3-oxopropanoate, and at least one further metabolite selected from (b) a ketone body selected from beta-hydroxybutyrate, acetoacetate, and combinations thereof, and (c) a medium chain fatty acid selected from octanoate, decanoate, and combinations thereof,

(ii) comparing the concentration of each of the metabolites determined in (i) with a corresponding reference value, and

(iii) diagnosing the patient as having cancer when the concentration of at least one of the metabolites is increased as compared to the corresponding reference value.

Generally, the reference value is, for each metabolite, the concentration of the metabolite determined in the biological sample obtained from a non-oncologic subject or group of patients.

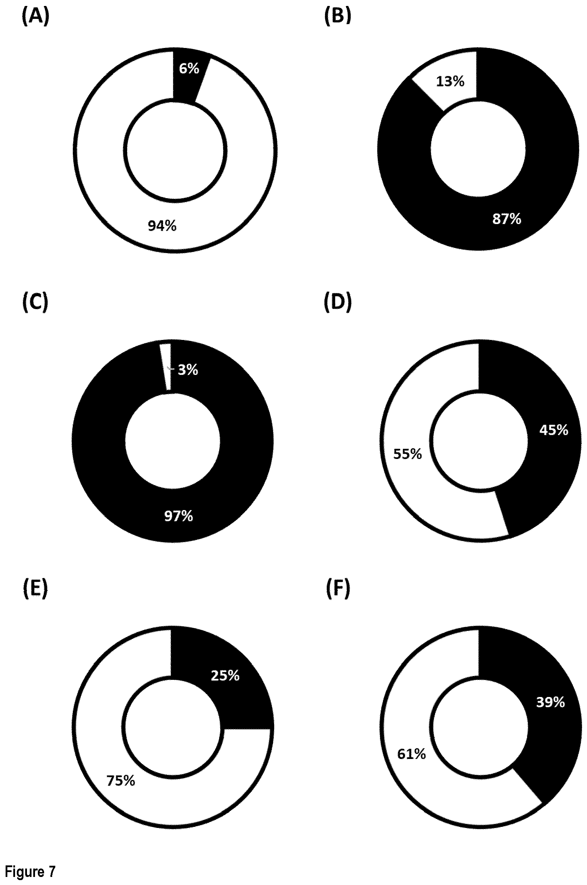

The diagnosis requires determining (a) 3-oxopropanoate, and at least (b) beta-hydroxybutyrate or acetoacetate, or (c) octanoate or decanoate. However, it suffices that one of these metabolites is increased when compared to its corresponding reference value to provide a cancer diagnosis. This is because most of the patients suffering from cancer have increased blood concentrations of at least one of the metabolites. As shown in Figure 7B, 87% blood samples of cancer patients (in this particular case, PDAC patients) included in the study had increased levels of at least one of the metabolites *versus* 6% in non-oncologic subjects. Many patients did not show increased levels of all metabolites but did have at least one of them. Looking for only one of the metabolites would correctly diagnose a significant proportion of cancer patients (Figure 7D-F). However, by looking for the signature, most cancer patients are diagnosed because they either have one of the metabolites included in the signature or they have more than one, or they have them all. Altogether, while each of the metabolites by itself already had discriminating capacity, determining the combination of biomarkers as disclosed in the present invention significantly improved the discrimination of cancer patients *versus* non-oncologic patients.

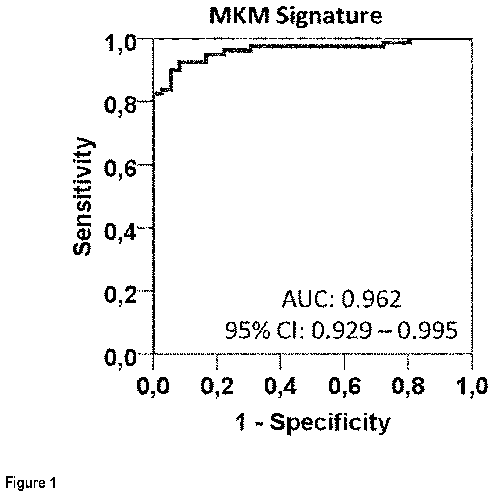

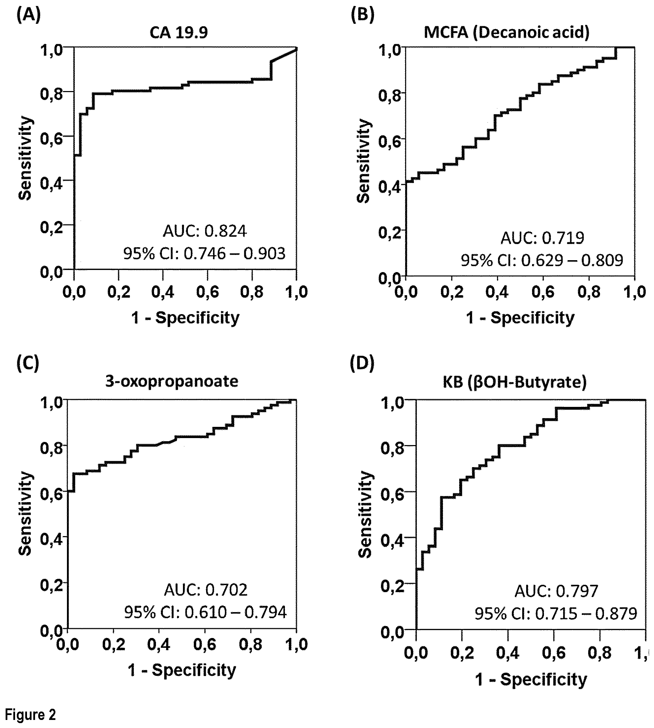

Importantly, the present diagnosis method provides surprisingly good predictive values for cancer diagnosis. As shown in the examples below, the metabolic signature of the invention successfully discerned between non-oncologic controls (including healthy controls and chronic pancreatitis patients) and patients having pancreatic cancer, in this particular case, PDAC, with a ROC-AUC of 0.939 (95% CI 0.899-0.979) (Figure 1). This result substantially improves the discriminating capabilities of CA 19.9 in the same samples (Figure 2). The method of the invention outperforms prior diagnostic methods, in particular prior methods that make use of biomarkers

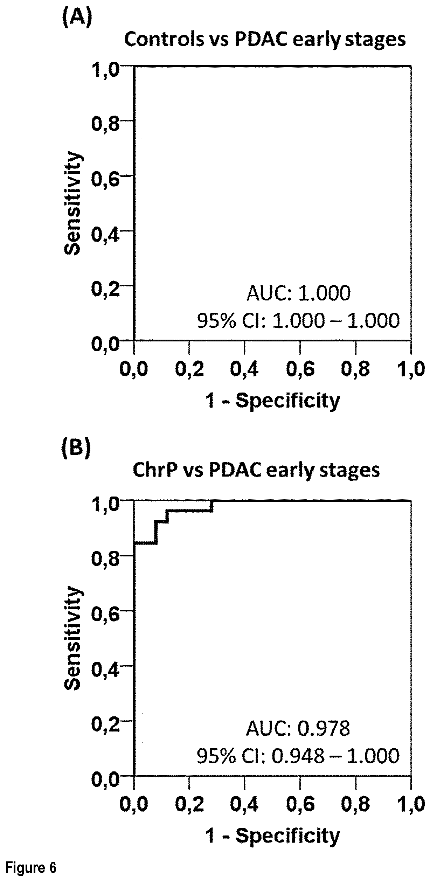

Interestingly, the metabolic signature of the invention was able to discriminate between patients having chronic pancreatitis and patients with pancreatic cancer. As shown in Figure 6B, a ROC-AUC of 0.978 (95% IC 0.948 to 1.000) was obtained when comparing early stage pancreatic cancer, in this particular case, PDAC, with chronic pancreatitis. This is an advantage for the clinical practice.

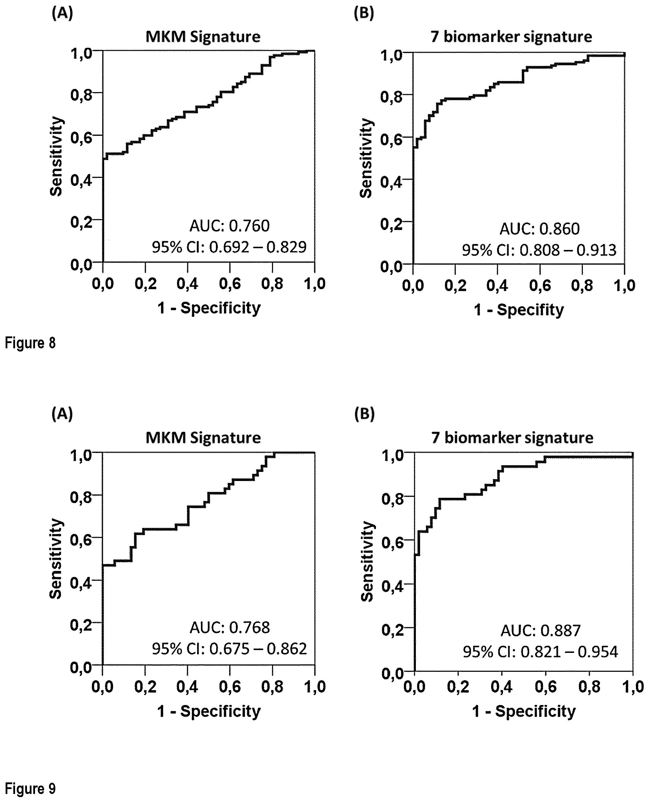

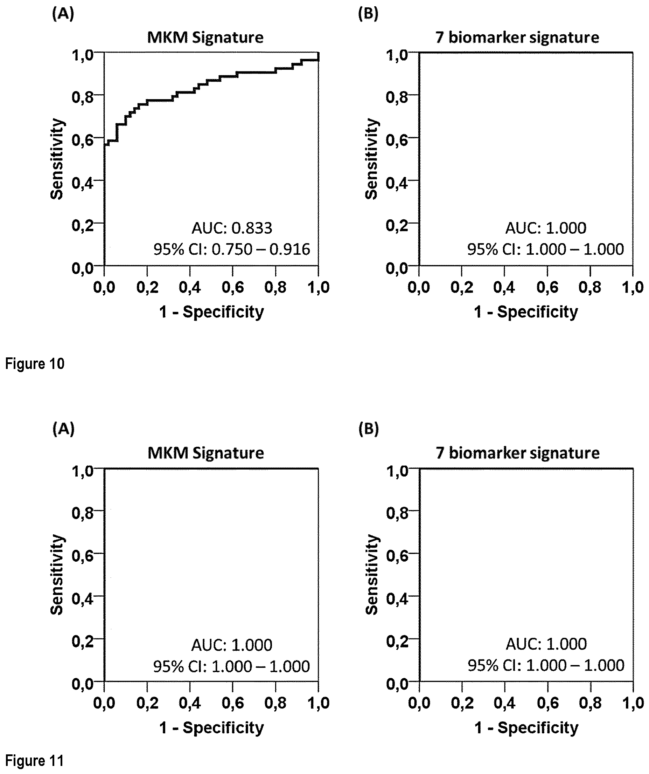

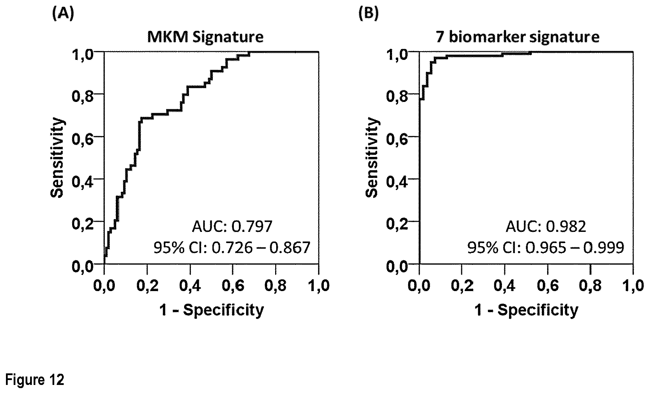

The metabolic signature of the invention also proved to distinguish non-oncologic patients from patients having other types of cancer with good sensitivities and specificities. For example, a ROC-AUC of 1.000 (95% IC 1.000-1.000) was obtained for gynecological cancer (Figure 11) and a ROC-AUC of 0.833 (95% IC 0.750-0.916) was obtained for esophageal cancer (Figure 10). Lung cancer could also be diagnosed by using the metabolic signature of the invention (AUC: 0.797; 95% CI: 0.726-0.867; Figure 12). Gastric cancer could also be diagnosed by using the metabolic signature of the invention (AUC: 0.760; 95% CI: 0.692-0.829; Figure 8). The results demonstrate that the metabolic signature of the invention significantly improves the diagnosis potential of previously described biomarkers for gastric cancer also.

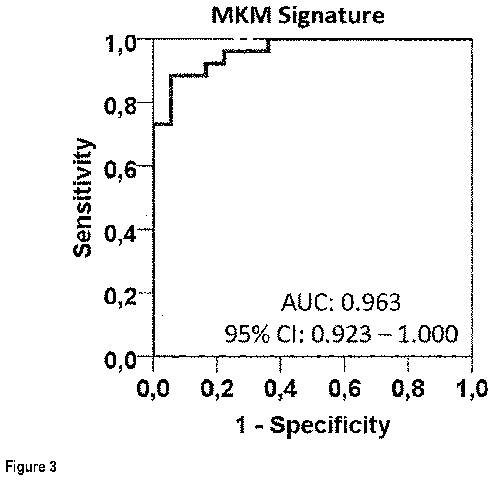

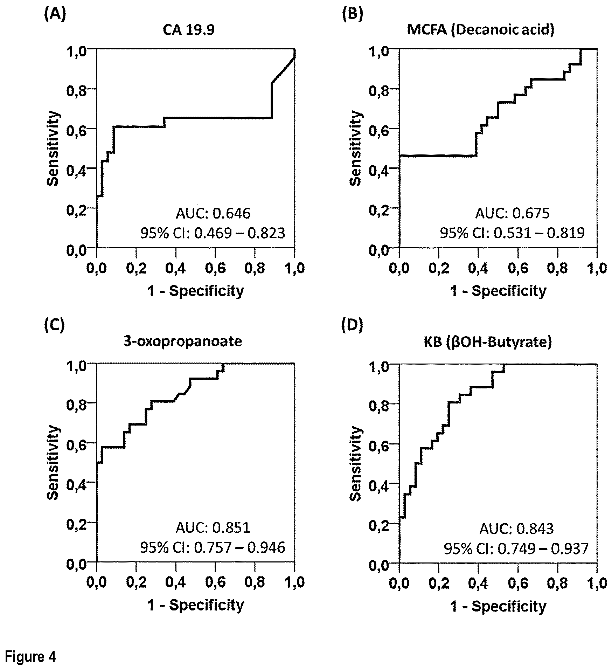

Importantly, the present method can also detect cancer in early stages, which is of outmost importance for patient managing. This is particularly relevant for some cancers, such as pancreatic or ovarian cancer, which are most often detected in late, incurable, stages, while early diagnosis would often be the only hope of a successful therapeutic intervention. When comparing early stage pancreatic cancer, in this particular case, PDAC samples, with controls (healthy subjects and patients suffering from pancreatitis), the resulting ROC-AUC value was 0.963 (95% IC 0.923-1.000) (Figure 3). This result substantially improves the discerning capabilities of CA 19.9 in the same samples (Figure 4).

The diagnosis method of the invention does not require providing a biopsied sample from the tumor. Instead, the diagnosis according to the invention may be performed, for example, by simply providing a blood sample, which is a far readily available biological sample. This method is also not invasive. Moreover, the present method is not limited to diagnosis of a particular type of cancer. The metabolic signature described herein has been found in different types of cancer. Consequently, several cancers may be diagnosed by the method of the invention.

Altogether, provided herein is a sensitive, specific, reliable, practical and cost-effective method for diagnosing cancer, including cancer in early stage. Moreover, the method of the invention may be used for cancer screening, including screening for early stage cancer.

A second aspect of the invention provides a kit of parts for diagnosing cancer as defined in the first aspect, said kit comprising: (i) means for determining the concentration of: (a) 3-oxopropanoate, and at least a further metabolite selected from (b) a ketone body selected from beta-hydroxybutyrate, acetoacetate and combinations thereof, and (c) a medium chain fatty acid selected from octanoate, decanoate and combinations thereof, and optionally means for determining the concentration of a further metabolite selected from (d) alpha-ketobutyrate, (e) gluconate, and combinations thereof,

(ii) reference values for the above metabolites, and
(iii) instructions for diagnosing cancer.

A third aspect refers to a combined use of (a) 3-oxopropanoate and at least one of (b) a ketone body selected from beta-hydroxybutyrate, acetoacetate and combinations thereof, or (c) a medium chain fatty acid selected from octanoate, decanoate and combinations thereof, as a biomarker for the diagnosis of cancer.

The invention further discloses a method for treating a subject suffering from cancer, said method comprising diagnosing cancer by an *in vitro* method as defined above and administering a medical regime for cancer to the patient if having cancer.

**Brief Description of Drawings**

Figure 1. ROC curve showing the potential of the diagnosis method of the invention (MKM signature containing the biomarkers 3-oxopropanoate, beta-hydroxybutyrate and decanoate) for the diagnosis of pancreatic cancer by using the whole PDAC cohort (patients diagnosed with both early and late stage PDAC).

Figure 2. ROC curves showing the individual potential of (A) CA19.9, (B) decanoate Medium Chain Fatty Acid (MCFA), (C) 3-oxopropanoate and (D) beta-hydroxybutyrate (KB)for the diagnosis of pancreatic cancer by using the whole PDAC cohort (both early and late diagnosed patients).

Figure 3. ROC curve showing the potential of the MKM signature containing the biomarkers 3-oxopropanoate, beta-hydroxybutyrate and decanoate for the early diagnosis of pancreatic cancer by using the early stage (stages I and IIA) PDAC cohort.

Figure 4. ROC curves showing the individual potential of (A) CA19.9, (B) MCFA, (C) 3-oxopropanoate and (D) KB for the early diagnosis of pancreatic cancer (stages I and IIA) by using the early stages PDAC cohort.

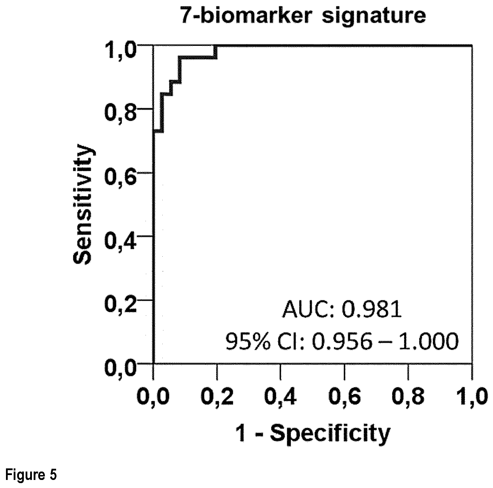

Figure 5. ROC curve showing the potential of the 7-biomarker signature containing the biomarkers 3-oxopropanoate, beta-hydroxybutyrate, decanoate, octanoate, acetoacetate, gluconate and alpha-ketobutyrate for the diagnosis of pancreatic cancer by using the whole PDAC cohort (patients diagnosed with both early and late stage PDAC).

Figure 6. ROC curve showing the potential of the 7-biomarker signature containing the biomarkers 3-oxopropanoate, beta-hydroxybutyrate, decanoate, octanoate, acetoacetate, gluconate and alpha-ketobutyrate for the early diagnosis of pancreatic cancer by using the early stage PDAC cohort. (A) Comparison between controls and early stages PDAC, (B) comparison between chronic pancreatitis and early stages PDAC.

Figure 7. Percentage of PDAC patients having increased plasma concentration of at least one of the biomarkers of the 7-metabolite signature. (A) 7-metabolite signature in non-oncologic (healthy+ pancreatitis) (N=36), (B) 7-metabolite signature in oncologic patients (PDAC) (N=80), (C) 7-metabolite signature + CA19.9 in oncologic (PDAC) patients (N=80). Percentage of PDAC patients having increased plasma concentrations of (D) 3-oxopropanoate, (E) one ketone body (beta-hydroxybutyrate or acetoacetate), (F) one medium chain fatty acid (octanoate or decanoate).

Figure 8. ROC curve showing the diagnosis potential of the selected biomarkers in gastric cancer. (A) MKM signature containing the biomarkers 3-oxopropanoate, beta-hydroxybutyrate and decanoate for the diagnosis of gastric cancer by using the gastric cancer cohort, (B) 7-metabolite signature containing the biomarkers 3-oxopropanoate, beta-hydroxybutyrate, decanoate, octanoate, acetoacetate, gluconate and alpha-ketobutyrate for the diagnosis of gastric cancer by using the gastric cancer cohort.

Figure 9. ROC curve showing the early-diagnosis potential of the selected biomarkers in gastric cancer. (A) MKM signature containing the biomarkers 3-oxopropanoate, beta-hydroxybutyrate and decanoate for the early-diagnosis of gastric cancer by using the gastric cancer cohort, (B) 7-metabolite signature containing the biomarkers 3-oxopropanoate, beta-hydroxybutyrate, decanoate, octanoate, acetoacetate, gluconate and alpha-ketobutyrate for the early-diagnosis of gastric cancer by using the gastric cancer cohort.

Figure 10. ROC curve showing the diagnosis potential of the selected biomarkers in esophageal cancer. (A) MKM signature containing the biomarkers 3-oxopropanoate, beta-hydroxybutyrate and decanoate for the diagnosis of esophageal cancer by using the esophageal cancer cohort, (B) 7-metabolite signature containing the biomarkers 3-oxopropanoate, beta-hydroxybutyrate, decanoate, octanoate, acetoacetate, gluconate and alpha-ketobutyrate for the diagnosis of esophageal cancer by using the esophageal cancer cohort.

Figure 11. ROC curve showing the diagnosis potential of the selected biomarkers in gynecological cancer. (A) MKM signature containing the biomarkers 3-oxopropanoate, beta-hydroxybutyrate and decanoate for the diagnosis of gynecological cancer by using the gynecological cancer cohort, (B) 7-metabolite signature containing the biomarkers 3-oxopropanoate, beta-hydroxybutyrate, decanoate, octanoate, acetoacetate, gluconate and alpha-ketobutyrate for the diagnosis of gynecological cancer by using the gynecological cancer cohort.

Figure 12. ROC curve showing the diagnosis potential of the selected biomarkers in lung cancer. (A) MKM signature containing the biomarkers 3-oxopropanoate, beta-hydroxybutyrate and decanoate for the diagnosis of lung cancer by using the lung cancer cohort, (B) 7-metabolite signature containing the biomarkers 3-oxopropanoate, beta-hydroxybutyrate, decanoate, octanoate, acetoacetate, gluconate and alpha-ketobutyrate for the diagnosis of lung cancer by using the lung cancer cohort

## Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "diagnosis" is known to the person skilled in the art. As used herein "diagnosis" is understood as becoming aware of a particular medical condition, complication; the determination of the nature of the condition; or the distinguishing of the condition from another. It refers both to the process of attempting to determine or identify the possible condition, and to the opinion reached by this process. A diagnosis, in the sense of diagnostic procedure, can be regarded as an attempt at classification of an individual's condition into separate and distinct categories that allow medical decisions about treatment and prognosis to be made.

"Early-stage" when referring to cancer is generally understood as the first stages in cancer disease, in particular, it is referred to stages IA, IB, IIA and IIB, more particularly to stages IA, IB, IIA.

The term "biomarker", as used herein, refers generally to a molecule, the concentration of which can be determined in a biological sample from a patient by standard methods (including those disclosed herein), and is predictive or denotes a condition of the patient from which it was obtained. In the present invention the biomarkers are one or more metabolites, or a combination of metabolites, the concentration of which may be determined, for example, by liquid chromatography coupled with tandem mass spectrometry.

The term "reference value" in the context of the present invention is to be understood as a predefined concentration of the biomarker, in this case, the metabolite, in a sample or group of samples. The samples are taken from a non-oncologic subject or group of subjects, e.g., a healthy subject. This value is used as a threshold to discriminate subjects wherein the condition, cancer, to be analysed is present from those wherein such condition is absent. The skilled person in the art, making use of the general knowledge, is able to choose the subject or group of subjects more adequate for obtaining the reference value. Methods for obtaining the reference value from the group of subjects selected are well known in the state of the art (Burtis C. A. et al., 2008, Chapter 14, section "Statistical Treatment of Reference Values"). As will be apparent to the skilled person, the reference values will be defined according to the purpose, target population for the diagnosis, balance between specificity and sensitibity, etc. In some embodiments, the reference value may also be a particular threshold value above which it is considered that the patient has cancer with predetermined specificity and sensitivity.

In most embodiments of the invention the concentration of the metabolite is measured as the weight of metabolite per volume of sample. When the biological sample is blood, plasma or serum, which are convenient biological samples for the method of the invention, the concentration is usually measured in ng metabolite/mL biological sample.

In its more general concept, the invention refers to an *in vitro* method for diagnosing cancer in a subject which comprises: (i) determining, in a sample obtained from the subject, the concentration of (a) 3-oxopropanoate, and at least one further metabolite selected from (b) a ketone body, and (c) a medium chain fatty acid; (ii) comparing the concentration of each of the metabolites determined in (i) with a corresponding reference value; and (iii) diagnosing the patient as having cancer when the concentration of at least one of the metabolites is increased as compared to the corresponding reference value.

Ketone bodies are selected from beta-hydroxybutyrate (IUPAC name: 3-hydroxybutanoate), acetoacetate (IUPAC name 3-oxobutanoate), acetone (IUPAC name: propan-2-one), and combinations thereof. In some embodiments, the ketone body is selected from beta-hydroxybutyrate, acetoacetate, and combinations thereof. Medium chain fatty acids are preferably selected from octanoate, decanoate, and combinations thereof.

In a particular embodiment of the first aspect of the invention, step (i) comprises determining the concentration of (a) 3-oxopropanoate, (b) a ketone body selected from beta-hydroxybutyrate, acetoacetate and combinations thereof, and (c) a medium chain fatty acid selected from octanoate, decanoate, and combinations thereof.

In one embodiment, step (i) comprises determining the concentration of decanoate. In a particular embodiment,

step (i) comprises determining the concentration of both decanoate and octanoate.

In one embodiment, step (i) comprises determining the concentration of beta-hydroxybutyrate. In a particular embodiment step (i) comprises determining the concentration of both beta-hydroxybutyrate and acetoacetate.

Thus, in a very particular embodiment of the invention, step (i) comprises determining 3-oxopropanoate, beta-hydroxybutyrate, acetoacetate, octanoate and decanoate.

As mentioned above, a significantly improved diagnosis as compared to prior methods that use biomarkers may be done by performing the method defined by the first aspect of the invention, which comprises determining: (a) 3-oxopropanoate, and (b) a ketone body selected from beta-hydroxybutyrate, acetoacetate and combinations thereof, or (c) a medium chain fatty acid selected from octanoate, decanoate and combinations thereof, in particular when determining (a), (b), and (c), more particularly when determining the concentration of 3-oxopropanoate, beta-hydroxybutyrate, acetoacetate, octanoate and decanoate. Nevertheless, the capacity to distinguish between non-oncologic and oncologic patients may be further improved by determining additional metabolites.

Appropriate metabolites for improving the method of the invention are alpha-ketobutyrate (IUPAC name: 2-oxobutanoate) and gluconate (IUPAC name: (2R,3S,4R,5R)-2,3,4,5,6-pentahydroxyhexanoate). Thus, in a more particular embodiment of the invention, step (i) further comprises determining the concentration of a metabolite selected from (d) alpha-ketobutyrate and (e) gluconate. In a still more particular embodiment, step (i) comprises determining 3-oxopropanoate, beta-hydroxybutyrate, acetoacetate, octanoate, decanoate, alpha-ketobutyrate, and gluconate.

In one embodiment, step (i) of the method further comprises determining the concentration of (d) alpha-ketobutyrate, (e) gluconate, or both. In a particular embodiment, the concentration of gluconate is determined. In a more preferred embodiment, the concentration of both gluconate and alpha-ketobutyrate is determined.

An "improved" diagnosis is understood as higher sensitivity and/or specificity which, as the skilled person would understand, may be determined by a higher ROC-AUC value. The inventors have found that the 7-metabolite signature is able to discriminate PDAC patients from controls with a receiver operating characteristic area under the curve (ROC-AUC) value of 0.97 (Figure 5). The 7-metabolite signature was further able to distinguish early-stage PDAC patients from healthy controls with a ROC-AUC value of 1.000 ((95% IC 1.000 to 1.000; Figure 6).

Additional biomarkers may be used to further enhance or complement the diagnosis of the invention. For example, the method may further comprise determining the concentration of another biomarker that has been disclosed for cancer diagnosis (or prognosis, or response to treatment) and comparing the determined concentration with a corresponding reference value. Thus, in one particular embodiment, step (i) of the method of the invention further comprises determining an additional marker related to cancer diagnosis or prognosis. Usually, in these cases, the patient may be diagnosed of having cancer with improved sensibility and/or specificity if said other biomarker is significantly increased or decreased when compared to its corresponding reference value. In a particular embodiment, the method of the invention further comprises determining CA19-9.

Appropriate additional biomarkers that can be used in the method of the invention may depend on the type of cancer. Non-limiting examples of biomarkers that may be useful for the method of the invention are provided below. For example, a biomarker selected from the group consisting of CA19-9, CA-242, CA-50, CA-195, CA 72-4, CEA and CA-125 may be used for diagnosing pancreatic cancer. In a particular embodiment, for diagnosing pancreatic cancer, the additional biomarker is CA19-9. A biomarker selected from the group consisting of Cytokeratin 19 fragment antigen 21-1 (Cyfra21-1), CA19-9, CA72-4, CEA and squamous cell carcinoma antigen (SCC-Ag) may be used for diagnosing esophageal cancer. In a particular embodiment, for diagnosing esophageal cancer, the additional biomarker is selected from CA19-9 and CEA. A biomarker selected from the group consisting of CA72-4, CA-50, alpha-fetoprotein, CA12-5, SLE, BCA-225, hCG and pepsinogen I/II may be used for diagnosing gastric cancer. In a particular embodiment, for diagnosing gastric cancer, the additional biomarker is selected from CEA and CA19-9. A biomarker selected from the group consisting of alpha-fetoprotein (AFP-L3), DCP/PVKA II, GPC3, GS, HSP70 (tissue), CK19, GP73, midkine, OPN, SCCA, Annexin A2 and FGF3/4 may be used for diagnosing liver cancer. In a particular embodiment, for diagnosing liver cancer, the additional biomarker is selected from alpha-fetoprotein (AFP-L3) and DCP/PVKA II. In another particular embodiment, for diagnosing cholangiocarcinoma, the additional biomarker is selected from CA19-9 and CA125. In another particular embodiment, for diagnosing ovarian cancer, the additional biomarker is CA125. In another particular embodiment, for diagnosing lung cancer, the additional biomarker is selected from NSE and CYFRA 21-1.

The sample obtained from the patient may be a tissue sample, or a biological sample (e.g., blood, plasma, serum, ascitic fluid, bronco-alveolar lavage, urine, breath, stool, CFS, saliva). In particular embodiments, the sample is a biological fluid. In more particular embodiments, the sample is selected from blood, plasma and serum. Early diagnosis is also facilitated by the invention, using devices such as the dry blood spots (DBS), which allow easy blood sample collection by simple pricking of a finger and mail transport to the reference center for its analysis without any special preservation conditions

The concentration of the metabolites may be determined by any analytical method available to the skilled person. In some embodiments, metabolites are determined using mass spectrometry. In some embodiments, liquid chromatography in combination with mass spectrometry is used. In particular embodiments, the concentration of the metabolites is determined by Liquid Chromatography with tandem mass spectrometry. The principle of tandem mass spectrometry is based on coupling mass spectrometers together in a series to analyze complex mixtures. The method uses two mass filters arranged sequentially with a collision cell between them. The filters can be used in static or scanning mode to select a particular mass-to- charge (m/z) ratio or m/z range. In the collision cell, the precursor ions collide with gas molecules and are fragmented into smaller ions referred to as product ions. Tandem mass spectrometers are versatile in that they can be operated using a variety of different scan modes depending on the clinical application. Exemplary mass spectrometry methodology is described in the examples.

Step (ii) of the diagnosis method of the invention comprises comparing the concentration of each of the metabolites determined in (i) with a corresponding reference value.

The reference value for each metabolite is the concentration of said metabolite determined in the biological sample obtained from a non-oncologic subject or group of subjects (controls). In some embodiments, the reference value for each metabolite is the concentration of said metabolite determined in the biological sample obtained from a healthy subject or group of subjects.

The reference value for each metabolite may be determined as described above. The inventors have determined exemplary reference values for each biomarker by determining the concentration in a control group of non-oncologic subjects. Based on these findings, reference values (reference concentrations) may be established. In one embodiment, the reference value for 3-oxopropanoate is in the range from 15 to 30 ng/mL, more particularly from 15 to 25 ng/mL. In one embodiment, the reference value for beta-hydroxybutyrate is in the range from 6000 to 10000 ng/mL, more particularly from 7000 to 9000 ng/mL. In one embodiment, the reference value for acetoacetate is in the range from 1000 to 3000 ng/mL, more particularly from 2000 to 3000 ng/mL. In one embodiment, the reference value for octanoate is in the range from 50 to 200 ng/mL, more particularly from 90 to 150 ng/mL. In one embodiment, the reference value for decanoate is in the range from 100 to 400 ng/mL, more particularly from 200 to 350 ng/mL. In one embodiment, the reference value for alpha-ketobutyrate is in the range from 120 to 200 ng/mL, in particular from 140 to 190 ng/mL. In a particular embodiment, the reference value for gluconate is in the range from 800 to 1500 ng/mL, more particularly from 900 to 1300 ng/mL.

The above values may provide a clear information for performing the comparing step (ii) as defined in the method of the invention. However, the skilled person may use any available method to establish said comparison.

Step (iii) of the diagnosis method of the invention comprises diagnosing the patient as having cancer when the concentration of at least one of the metabolites is increased as compared to the corresponding reference value.

In one embodiment, the patient is diagnosed with cancer if at least one of the metabolites is increased at least 2-fold when compared to the corresponding reference value. In a particular embodiment, the increase is at least 3-fold. In another particular embodiment, the increase is at least 4-fold. In another particular embodiment, the increase is at least 5-fold. In another particular embodiment, the increase is at least 6-fold.

In one embodiment, the patient is diagnosed with cancer if at least one of the metabolites is increased at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150%, at least 200%, at least 300%, at least 400%, at least 500%, or at least 600% or more higher than the reference value.

Based on the reference values, threshold values may be provided for each of the metabolites. The "threshold value", also referred to as "cut-off value", for a metabolite is understood as a concentration value above which the patient is diagnosed as having cancer (according to the method of the invention) with good sensitivity and specificity. Thus, in some embodiments, the reference value is a threshold value. In these embodiments, step (ii) of the method of the invention comprises comparing the concentration of each of the metabolites determined in (i) with a corresponding threshold value and step (iii) comprises diagnosing the patient as having cancer when the concentration of at least one of the metabolites is increased as compared to the corresponding threshold value.

In one embodiment, the threshold value for 3-oxopropanoate is from 50 ng/mL to 500 ng/ml, in particular from 70 to 300 ng/mL, more particularly from 150 to 300 ng/mL, and even more particularly, around 250 ng/mL.

In one embodiment, the threshold value for beta-hydroxybutyrate is from 25000 to 70000 ng/mL, in particular from 30000 to 60000 ng/mL, more particularly from 40000 to 55000 ng/mL, and even more particularly, around 50000 ng/mL.

In one embodiment, the threshold value for acetoacetate is from 9000 to 15000 ng/mL, in particular from 10000 to 13500 ng/mL, more particularly from 11500 to 13000 ng/mL, and even more particularly, around 12500 ng/mL.

In one embodiment, the threshold value for octanoate is from 750 to 4000 ng/mL, in particular from 1000 to 3000 ng/mL, more particularly from 1500 to 2500 ng/mL, and even more particularly, around 2000 ng/mL.

In one embodiment, the threshold value for decanoate is from 1300 to 4500 ng/mL, in particular from 1500 to 4000 ng/mL, more particularly from 2000 to 3500 ng/mL, and even more particularly, around 3000 ng/mL.

In one embodiment, the threshold value for alpha-ketobutyrate is from 400 to 1000 ng/mL, in particular from 500 to 900 ng/mL, more particularly from 600 to 800 ng/mL, and even more particularly, around 700 ng/mL.

In one embodiment, the threshold value for gluconate is from 3000 to 6500 ng/mL, in particular from 3500 to 6000 ng/mL, more particularly from 4000 to 5500 ng/mL, and even more particularly, around 5000 ng/mL.

In one embodiment, the threshold value for octanoate+decanoate is from 2000 to 5000 ng/mL, in particular from 3000 to 4500 ng/mL, more particularly around 3500 ng/mL, and even more particularly, around 4000 ng/mL.

In one embodiment, the threshold value for beta-hydroxybutyrate + acetoacetate is from 40000 to 60000 ng/mL, in particular around 45000 ng/mL, more particularly around 50000 ng/mL, and even more particularly, around 55000 ng/mL.

The method of the invention requires that at least one the metabolites determined in step (i) is increased in order to diagnose cancer. However, it is of course possible that two, three, four, five, six or seven metabolites are increased when compared with their corresponding reference values. In a particular embodiment, at least (a), i.e., at least 3-oxopropanoate, is increased as compared to the corresponding reference value. In a more particular embodiment at least (a) and (b) are increased. In another more particular embodiment at least (a) and (c) are increased. In an even more particular embodiment, (a), (b) and (c) are increased. In a still more particular embodiment, 3-oxopropanoate, beta-hydroxybutyrate, acetoacetate, octanoate, decanoate, and, optionally, alpha-ketobutyrate and gluconate are increased. The metabolite (a) and at least one of (b) or (c) are determined. However, as explained above, it is enough that only one of (a), (b), or (c) is higher than a corresponding reference value to differentiate between oncologic and non-oncologic patients.

As outlined above, the method of the invention outperforms prior diagnostic methods, in particular prior methods that make use of biomarkers. The performance of the diagnosis method, in this case, the ability to discriminate between oncologic and non-oncologic patients, may be defined by its sensitivity and specificity, which may be in turn determined by a receiver operating characteristic (ROC) curve and by calculating the corresponding area under the curve (AUC). In one embodiment, the method for diagnosing cancer of the invention is characterized by a AUC-ROC value in the range from 0.750 to 1.000. In a particular embodiment, the method is characterized by a AUC-ROC value in the range above 0.800. In a more particular embodiment, the method is characterized by a AUC-ROC value in the range above 0.850. In an even more particular embodiment, the method is characterized by a AUC-ROC value above 0.900. In an even more particular embodiment, the method is characterized by a AUC-ROC value above 0.950, more particularly above 0.960, more particularly above 0.970, more particularly above 0.980.

The above AUC-ROC values determine the sensitivity and specificity of the method. In a particular embodiment, the sensitivity of the method of the invention ranges from 75.00% to 100.00% or from 75.00% to 99.00%. In a more particular embodiment, the sensitivity of the method of the invention is above 80.00%. In an even more particular embodiment, the sensitivity of the method of the invention is above 85%. In an even more particular embodiment the sensitivity is above 87%, for example 88%, 89%, 90%, 91%, 92%, 93%, 94%, 95%, 96%, 97%, 98% or 99%. In a particular embodiment, the specificity of the method of the invention ranges from 75.00% to 100.00% or from 75.00% to 99.00%. In a more particular embodiment, the specificity of the method of the invention is above 80.00%. In an even more particular embodiment, the specificity of the method of the invention is above 85%. In an even more particular embodiment the specificity is above 87%, for example 88%, 89%, 90%, 91%, 92%, 93%, 94%, 95%, 96%, 97%, 98% or 99%.

As outlined above, the method of the invention may be used for diagnosing cancer in early stages. It cannot be sufficiently stressed how important it is to have tools for diagnosing early stage cancer. It is well known that early diagnosis increases the chances of therapeutic success enormously. In some cases, such as pancreatic cancer, early diagnosis is often the only hope of a successful therapeutic intervention. Thus, in a particular embodiment, the method of the invention is for diagnosing early-stage cancer, in particular, cancer in stages IA, IB and IIA. All the embodiments disclosed above for cancer diagnosis (in general), including AUC-ROC values, sensitivity and specificity values, also apply to the method of the invention when diagnosing early-stage cancer.

The method of the invention may be used to diagnose most types of cancers. In one embodiment, the method of the invention is for diagnosing solid cancers. Solid cancers are defined as abnormal masses of tissue that usually do not contain cysts or liquid areas. They grow in organ systems such as pancreas, stomach, liver, lung, esophagus, breast or ovaries and can occur anywhere in the body. Examples of solid tumors are sarcomas, carcinomas, and lymphomas. In particular embodiments, the method of the invention is for diagnosing a cancer selected from the group consisting of pancreatic cancer, gastric cancer, esophageal cancer, gynecological cancer, breast cancer, colorectal cancer, and lung cancer. In more particular embodiments, the method of the invention is for diagnosing pancreatic cancer, gastric cancer, esophageal cancer, lung cancer or gynecological cancer. In an even more particular embodiment, the method of the invention is for diagnosing pancreatic cancer, for example pancreatic ductal adenocarcinoma. It is a very significant advantage if the method of the invention that cancer, for example, pancreatic cancer, can be diagnosed in an early stage.

Further provided is a method for treating a subject suffering from cancer, said method comprising diagnosing cancer by an *in vitro* method as defined above and administering an appropriate therapeutic regime for cancer to the patient if diagnosed with cancer. For example, the therapeutic regime may be surgical resection, radiotherapy, chemotherapy, immunotherapy, hormonal therapy, hyperthermia, photodynamic therapy, stem cell transplant or combinations thereof. Thus, in particular embodiments the method is for diagnosing early-stage cancer, so it provides useful information for the clinician to choose the therapeutic regime to be administered or indicates the one that is best suited or only possible for patients having cancer in early stages. In particular, the only curative therapeutic regime may be surgical resection in the case of pancreatic, gastric, esophageal and lung cancer if they are diagnosed at an early stage. This is especially relevant in many cases for which no other effective therapy is available, such as pancreatic cancer. For example, when the diagnosis is of early-stage PDAC, the patient may be treated with curative intention by radical resection with tumor free margins.

Also disclosed is a kit of parts comprising means for determining the concentration of (a) 3-oxopropanoate, and another metabolite selected from (b) a ketone body selected from beta-hydroxybutyrate, acetoacetate and combinations thereof, and (c) a medium chain fatty acid selected from octanoate, decanoate and combinations thereof, and optionally means for determining the concentration of a further metabolite selected from (d) alpha-ketobutyrate, (e) gluconate, and combinations thereof. Said means may include reagents and buffers for sample treatment, standards for calibration, elution buffers, etc. The kit may also comprise reference values for the above metabolites, and it often comprises instructions enabling the comparison of the determined biomarkers with reference the values in order to make the diagnosis. In most cases the kit comprises information about threshold values, calibration, sample treatment, assay conditions, etc. The kit may also contain a blood extraction device, for example, a dry blood spots (DBS), which allows easy blood sample collection by simple pricking of a finger.

The present invention further provides for the use of the kits as defined above for the diagnosis of cancer according to the method of the invention.

The method of the invention may be automated in order to provide a diagnosis result. Thus, the invention also provides a system for determining the diagnosis of cancer in a patient comprising data processing means, said data processing means been configured:

- to assess in a test sample obtained from the patient the concentration of (a) and either (b) or (c) or both (b) and (c), and optionally a further metabolite selected from (d), (e) or both (d) and (e);
- to determine whether said concentrations are significantly up-regulated as compared corresponding reference values; and
- to determine the diagnosis of cancer by evaluating the result of the previous determination.

The *in vitro* method of the invention generally provide for determining the diagnosis of cancer in a patient and for recommending an appropriate medical regime for the treatment of said patient. In some embodiments, said method may further comprise the steps of (i) collecting the diagnostic information, and (ii) saving the information in a data carrier.

In the sense of the invention a "data carrier" is to be understood as any means that contain meaningful information data for determining the diagnosis of cancer, in particular, of early-stage cancer, in a patient and/or recommending an appropriate medical regime, such as paper. The carrier may also be any entity or device capable of carrying the prognosis data or information for recommending an appropriate therapy. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the diagnosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

For completeness, the present description is also disclosed in the following numbered embodiments.

1. An *in vitro* method for diagnosing cancer in a subject, said method comprising:

(i) determining, in a sample obtained from the subject, the concentration of (a) 3-oxopropanoate, and at least one further metabolite selected from (b) a ketone body, and (c) a medium chain fatty acid;
(ii) comparing the concentration of each of the metabolites determined in (i) with a corresponding reference value; and
(iii) diagnosing the patient as having cancer when the concentration of at least one of the metabolites is increased as compared to the corresponding reference value.

2. The *in vitro* method for diagnosing cancer in a subject according to the preceding embodiment, said method comprising the steps of:

(i) determining, in a sample obtained from the subject, the concentration of (a) 3-oxopropanoate, and at least one further metabolite selected from (b) a ketone body selected from beta-hydroxybutyrate, acetoacetate, and combinations thereof, and (c) a medium chain fatty acid selected from octanoate, decanoate, and combinations thereof;

(ii) comparing the concentration of each of the metabolites determined in (i) with a corresponding reference value; and

(iii) diagnosing the patient as having cancer when the concentration of at least one of the metabolites is increased as compared to the corresponding reference value.

3. The *in vitro* method according to any one of the preceding embodiments, wherein step (i) comprises determining (a), (b) and (c).

4. The *in vitro* method according to any one of the preceding embodiments, wherein step (i) comprises determining the concentration of decanoate.

5. The *in vitro* method according to any one of the preceding embodiments, wherein step (i) comprises determining the concentration of both decanoate and octanoate.

6. The *in vitro* method according to any one of the preceding embodiments, wherein step (i) comprises determining the concentration of beta-hydroxybutyrate.

7. The *in vitro* method according to any one of the preceding embodiments, wherein step (i) comprises determining the concentration of both beta-hydroxybutyrate and acetoacetate.

8. The *in vitro* method according to any one of the preceding embodiments, wherein step (i) comprises determining 3-oxopropanoate, beta-hydroxybutyrate, acetoacetate, octanoate and decanoate.

9. The *in vitro* method according to any one of the preceding embodiments, wherein at least one metabolity is increased as compared to the corresponding reference value, in particular, said metabolite is 3-oxopropanoate.

10. The *in vitro* method according to any one of the preceding embodiments, wherein at least two metabolites are increased as compared to the corresponding reference values.

11. The *in vitro* method according to any one of the preceding embodiments, wherein at least three, four or five metabolites are increased as compared to the corresponding reference values.

12. The *in vitro* method according to any one of the preceding embodiments, wherein step (i) further comprises determining the concentration of (d) alpha-ketobutyrate.

13. The *in vitro* method according to any one of the preceding embodiments, wherein step (i) further comprises determining the concentration of (e) gluconate.

14. The *in vitro* method according to any one of the preceding embodiments, wherein step (i) comprises determining 3-oxopropanoate, beta-hydroxybutyrate, acetoacetate, octanoate, decanoate, alpha-ketobutyrate, and gluconate.

15. The *in vitro* method according to any one of the preceding embodiments, wherein the reference value for each metabolite is the concentration of said metabolite determined in the biological sample obtained from a non-oncologic subject or group of subjects.

16. The *in vitro* method according to any one of the preceding embodiments, wherein the increase is at least 3-fold.

17. The *in vitro* method according to the preceding embodiment, wherein the increase is at least 4-fold, in particular at least 6-fold.

18. The *in vitro* method according to any one of embodiments 1-15, wherein the reference value is a threshold value.

19. The *in vitro* method according to the preceding embodiment, wherein the threshold value for 3-oxopropanoate is from 150 to 300 ng/mL, in particular, around 250 ng/mL.

20. The *in vitro* method according to any one of embodiments 18-19, wherein the threshold value for beta-hydroxybutyrate is from 40000 to 55000 ng/mL, in particular, around 50000 ng/mL.

21. The *in vitro* method according to any one of embodiments 18-20, wherein the threshold value for acetoacetate is from 11500 to 13000 ng/mL, in particular, around 12500 ng/mL.

22. The *in vitro* method according to any one of embodiments 18-21, wherein the threshold value for octanoate is from 1500 to 2500 ng/mL, in particular, around 2000 ng/mL.

23. The *in vitro* method according to any one of embodiments 18-22, wherein the threshold value for decanoate is from 2000 to 3500 ng/mL, in particular, around 3000 ng/mL.

24. The *in vitro* method according to any one of embodiments 18-23, wherein the threshold value for alpha-ketobutyrate is from 600 to 800 ng/mL, in particular, around 700 ng/mL.

25. The *in vitro* method according to any one of embodiments 18-24, wherein the threshold value for gluconate is from 4000 to 5500 ng/mL, in particular, around 5000 ng/mL.

26. The *in vitro* method according to any one of embodiments 18-25, wherein the threshold value for octanoate+decanoate is from 3000 to 4500 ng/mL, in particular, around 4000 ng/mL.

27. The *in vitro* method according to any one of embodiments 18-26, wherein the threshold value for beta-hydroxybutyrate + acetoacetate is from 40000 to 60000 ng/mL, in particular, around 55000 ng/mL.

28. The *in vitro* method according to any one of the preceding embodiments, wherein the method is characterized by a receiver operating characteristic area under the curve value in the range from 0.700 to 1.000, in particular in the range from 0.800 to 1.000.

29. The *in vitro* method according to the preceding embodiment, wherein the method is characterized by a receiver operating characteristic area under the curve value in the range from 0.900 to 1.000, in particular in the range from 0.950 to 1.000.

30. The *in vitro* method according to any one of the preceding embodiments, wherein the sensitivity of the method of the invention is above 70.00%.

31. The *in vitro* method according to the preceding embodiment, wherein the sensitivity of the method of the invention is above 80.00%.

32. The *in vitro* method according to the preceding embodiment, wherein the sensitivity of the method of the invention is above 90.00%.

33. The *in vitro* method according to the preceding embodiment, wherein the sensitivity of the method of the invention is above 95.00%.

34. The *in vitro* method according to the preceding embodiment, wherein the sensitivity of the method of the invention is above 98.00%.

35. The *in vitro* method according to the preceding embodiment, wherein the specificity of the method of the invention is above 70.00%.

36. The *in vitro* method according to the preceding embodiment, wherein the specificity of the method of the invention is above 80.00%.

37. The *in vitro* method according to the preceding embodiment, wherein the s specificity of the method of the invention is above 90.00%.

38. The *in vitro* method according to the preceding embodiment, wherein the specificity of the method of the invention is above 95.00%.

39. The *in vitro* method according to any the preceding embodiment, wherein the specificity of the method of the invention is above 98.00%.

40. The *in vitro* method according to any one of the preceding embodiments, wherein the subject is diagnosed as having early stage cancer, in particular, cancer in stages IA, IB or IIA.

41. The *in vitro* diagnosis according to any one of the preceding embodiments, that is for diagnosing a solid tumor.

42. The *in vitro* diagnosis according to the preceding embodiment, wherein the cancer is selected from the group consisting of sarcomas, carcinomas, and lymphomas.

43. The *in vitro* diagnosis according to the preceding embodiment, wherein the cancer is selected from the group consisting of pancreatic cancer, gastric cancer, esophageal cancer, gynecological cancer, breast cancer, colorectal cancer, and lung cancer, in particular pancreatic cancer, more particularly pancreatic ductal adenocarcinoma.

44. The *in vitro* diagnosis according to any one of the preceding claims, wherein the sample is selected from blood, plasma, serum, ascitic fluid, bronco-alveolar lavage, urine, breath, stool, CFS, and saliva

45. The *in vitro* diagnosis according to the preceding embodiment, wherein the sample is selected from blood, plasma and serum.

46. The *in vitro* diagnosis according to any one of the preceding embodiments, wherein the amount of metabolite is determined by Liquid Chromatography with tandem mass spectrometry.

47. A kit of parts comprising:

(i) means for determining the concentration of (a) 3-oxopropanoate, and at least one other metabolite selected from (b) a ketone body selected from beta-hydroxybutyrate, acetoacetate and combinations thereof, and (c) a medium chain fatty acid selected from octanoate, decanoate and combinations thereof, and optionally means for determining the concentration of a further metabolite selected from (d) alpha-ketobutyrate, (e) gluconate, and combinations thereof,
(ii) optionally, reference values for the above metabolites, and
(iii) instructions for diagnosing cancer.

48. The kit of parts according to the preceding embodiment, that comprises means for determining the concentration of 3-oxopropanoate, beta-hydroxybutyrate, acetoacetate, octanoate, and decanoate.

49. The kit of parts according to the preceding embodiment, additionally comprising means for determining the concentration of alpha-ketobutyrate and gluconate.

50. A kit of parts as defined in any one of embodiments 47-49 for diagnosing cancer as defined in any one of embodiments 1-46.

51. Combined use of (a) 3-oxopropanoate and at least one of (b) a ketone body selected from beta-hydroxybutyrate, acetoacetate and combinations thereof, or (c) a medium chain fatty acid selected from octanoate, decanoate and combinations thereof, as a biomarker for the diagnosis of cancer.

52. Combined use of 3-oxopropanoate, beta-hydroxybutyrate, acetoacetate, octanoate, and decanoate , as a biomarker for the diagnosis of cancer.

53. Combined use of 3-oxopropanoate, beta-hydroxybutyrate, acetoacetate, octanoate, decanoate, alpha-ketobutyrate and gluconate as a biomarker for the diagnosis of cancer.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

**Examples**

**1.** Analysis of the metabolic signature in cancer patients by liquid chromatography-tandem mass spectrometry (LC-MS/MS)

1.1. Methods

Patients

Pancreatic cancer. The Pancreatic Cancer Cohort comprised 80 PDAC patients, 25 chronic pancreatitis (ChrP) patients and 11 healthy controls (HC), was collected at Hospital Universitari Doctor Josep Trueta (Girona, Spain). After diagnosis samples were classified as shown in Table 1. The overall resection rate was 89%. Serum samples were collected the day before surgery for patients with Stages I/II and the day before starting chemotherapy for patients with Stages III/IV PDAC.

**Table 1**. Patient demographics of the Pancreatic Cancer.

| PDAC AJCC STAGE | n= | Pancreatic Cancer Cohort | | Median age years (range) |
| | | Sex | | |
| | | Male | Female | |
|---|---|---|---|---|
| I | 9 | 5 | 4 | 73 (56-80) |
| IIA | 17 | 9 | 8 | 70 (55-79) |
| IIB | 25 | 7 | 18 | 67 (49-81) |
| III | 13 | 10 | 3 | 64 (48-73) |
| IV | 16 | 9 | 7 | 67 (30-81) |
| ChrP | 25 | 19 | 6 | 55 (39-75) |
| HC | 11 | 6 | 5 | 62 (44-75) |

Gastric cancer. The Gastric Cancer Cohort comprised sera from 127 patients diagnosed with adenocarcinomas from the stomach or the gastroesophageal junction (Siewert type III) who underwent surgery during the period 2004-2016 at the Gastro-Intestinal Surgery Department from Hospital del Mar, (Barcelona, Spain) and 52 healthy controls (HC) collected the Hospital del Mar (n=41) and Hospital Universitari Doctor Josep Trueta (n=11, Girona, Spain). After diagnosis samples were classified as shown in Table 2. Serum samples were collected the day before starting neoadjuvant treatment or before surgery in the case of the patients who did not receive it.

Table 2. Patient demographics of the Gastric Cancer Cohort

| GC AJCC STAGE | n= | Gastric Cancer Cohort | | Median age years (range) |
| | | Sex | | |
| | | Male | Female | |
|---|---|---|---|---|
| 0 | 1 | 1 | | 73 |
| IA | 15 | 10 | 5 | 73 (69-86) |
| IB | 16 | 8 | 8 | 78 (49-89) |
| IIA | 16 | 8 | 8 | 78 (32-89) |
| IIB | 15 | 9 | 6 | 73 (38-86) |
| IIIA | 12 | 6 | 6 | 75 (59-84) |
| IIIB | 19 | 12 | 7 | 73 (52-90) |
| IIIC | 20 | 14 | 6 | 70 (42-90) |
| IV | 13 | 6 | 7 | 74 (50-88) |
| HC | 52 | 39 | 13 | 63 (27-81) |

Esophageal cancer. The Esophageal Cancer Cohort comprised sera from 52 patients diagnosed with adenocarcinomas or squamous cell carcinomas from the esophagus or the gastroesophageal junction (Siewert types I and II) who underwent surgery during the period 2006-2019 at the gastro-Intestinal Surgery Department from Hospital del Mar, (Barcelona, Spain) and 50 healthy controls (HC) collected at the Blood Bank from Hospital Vall d'Hebrón (Barcelona). Serum samples were collected the day before starting the neoadjuvant treatment.

Table 3. Patient demographics of the Esophageal Cancer Cohort

| | Esophageal Cancer Cohort | | | |
|---|---|---|---|---|
| EC AJCC STAGE | n= | Sex | | Median age years (range) |
| | | Male | Female | |
| I | 6 | 6 | 0 | 62 (55-79) |
| IIA | 5 | 2 | 3 | 75 (41-81) |
| IIB | 4 | 4 | 0 | 57 (42-75) |
| III | 28 | 24 | 4 | 67 (45-82) |
| IVA | 3 | 2 | 1 | 53 (40-65) |
| IVB | 3 | 3 | 0 | 61 (45-75) - |
| UNK | 3 | - | - | |
| HC | 50 | 33 | 17 | 62 (51-69) |

Small Cell Lung Cancer Cohort. The Small Cell Lung Cancer (SCLC) Control comprised sera from 98 patients diagnosed with small cell lung cancer at the Medical Oncology Department at Hospital del Mar (Barcelona, Spain) and 50 HC collected at the Blood Bank from Hospital Vall d'Hebrón (Barcelona).

Table 4. Patient demographics of the SCLC Cancer Cohort

| | SCLC Cohort | | | |
|---|---|---|---|---|
| EC AJCC STAGE | n= | Sex | | Median age years (range) |
| | | Male | Female | |
| I | 3 | 1 | 2 | 76 (76-81) |
| III | 3 | 3 | 0 | 77 (57-78) |
| III | 30 | 17 | 13 | 67 (45-87) |
| IV | 62 | 48 | 14 | 68 (51-88) |
| HC | 50 | 33 | 17 | 61 (51-69) |

Gynecological cancer. The Gynecological Cancer Cohort comprised sera from 10 patients diagnosed with endometrial (N=4), ovarian (N=2), cervical (N=2) and uterine (N=1) cancer and 6 HC sera collected at Hospital del Mar.

Table 5. Patient demographics of the Gynecological Cancer Cohort.

| | Gynecological Cancer Cohort | | | |
|---|---|---|---|---|
| cancer type | Stage | | | Median age years (range) |
| | I | II | III | |
| endometrial | 4 | - | 1 | 59 (43-73) |
| ovarian | - | - | 2 | 68 (67-68) |
| cervical | 1 | 1 | - | 51 (46-57) |
| uterine | 1 | - | - | 60 |
| HC         n=6 | NA | NA | NA | 56 (45-79) |

Sample treatment

Blood was collected by venipuncture into Vacutainer[R] gel tubes without anticoagulant (serum samples) or with dipotassium-EDTA (plasma samples). Blood samples were allowed to cloth for 30 minutes and centrifuged at 1200xg for 10 minutes at 4°C. Serum and plasma samples were aliquoted and stored at -80°C. All samples were collected and processed using the same protocol.

Serum and plasma samples obtained from cancer patients and control population were treated based on a method reported in Gomez-Gomez *et al* (Gómez-Gómez et al, Journal of Pharmaceutical and Biomedical Analysis. 2022 Jan; 208, 114450). First, proteins were precipitated by adding 40 $\mu$L of cold methanol to 10 $\mu$L of plasma (5-fold plasma dilution). Then, 25 $\mu$L of the diluted sample were mixed with 30 $\mu$L of internal standard working solution consisting on a methanolic mixture containing octanoate-13C4, $\beta$-hydroxybutyrate-d4, Lactate-d3 and pyruvate-13C3 all at 0.5 $\mu$g/mL. Targeted metabolites were derivatized using 500 $\mu$L of o-benzyl hydroxylamine and 500 $\mu$L of N-(3-Dimethylaminopropyl)-N'-ethylcarbodiimide hydrochloride at 1 M) during 1 h at room temperature. Derivatization reaction was stopped by addition of 1 mL of milliQ water. Analytes were extracted by a liquid-liquid extraction after addition of 4 mL of ethyl acetate and vortexing at maximum power during 1 minute. After centrifugation (5 minutes, 3800 rpm), the organic layer was separated and dried under a nitrogen stream in a water bath at 40 °C. The extracts were reconstituted in 300 $\mu$L of water: methanol (1/1). Finally, 10 $\mu$L were injected into the liquid chromatography-tandem mass spectrometry (LC-MS/MS) system.

LC-MS/MS

The LC-MS/MS system was an Acquity UPLC system (Waters Associated, Milford, MA, USA) coupled to a triple quadrupole (Xevo TQ-S) mass spectrometer (Waters Associated) provided with an orthogonal Z-spray-electrospray interface (ESI).

The LC chromatography separation was performed using an Acquity BEH C18 column (100 mm x 2.1 mm i.d., 1.7 $\mu$m) (Waters Associates) with a flow rate of 0.3 mL/min at 55 °C. The selected mobile phases were water-ammonium formate (1 mM)-formic acid (0.01%) as mobile phase A and methanol-ammonium formate (1 mM)-formic acid (0.01%) as mobile phase B.

The MS/MS detection was achieved by using the positive ionization mode. The MS used argon as collision gas and nitrogen as drying and nebulizing gas. The desolvation gas flow was set at 1200 L/h and the cone gas at 50 L/h. A capillary voltage of 3 kV was selected for the MS detection. Nitrogen desolvation temperature and source temperature were set to 600 °C and 150 °C, respectively.

A targeted LC-MS/MS method was developed for the quantification of the metabolites of interest. The approach used a 8.75 minutes chromatographic gradient in which the percentage of the methanolic mobile phase (B) linearly increased as follows: 1 min, 20%; 2 min, 30%; 2.01 min, 35%; 2.75 min, 45%; 2.76 min, 65%; 6.75 min, 85%; 6.76 min, 95%; 7.75 min, 95%; 7.76 min, 20%; 8.75 min, 20%. Determination of the metabolites was performed by a selected reaction monitoring method including two ion transitions per metabolite. The main characteristics of the method are shown in Table 6. Quantification was performed by external calibration comparing the response (area of the analyte/area of the internal standard) of the metabolites in the samples with the response obtained for calibration standards with known concentrations.

Table 6. SRM conditions for the targeted determination of metabolites

| Metabolite | Retention time (min) | Precursor ion (m/z) | Cone voltage (V) | Collision energy (V) | Product ion (m/z) |
|---|---|---|---|---|---|
| Octanoate | 5.52 | 250 | 20 | 20 | 124[a] |
|  |  |  | 20 | 10 | 91 |
| Decanoate | 6.65 | 278 | 20 | 20 | 124[a] |
|  |  |  | 20 | 10 | 91 |
| Acetoacetate | 4.89 | 313 | 20 | 10 | 181[a] |
|  |  |  | 20 | 20 | 91 |
| $\beta$-hydroxybutyrate | 3.45 | 210 | 20 | 10 | 124[a] |
|  |  |  | 20 | 20 | 91 |
| 3-oxopropanoate | 4.67 | 299 | 20 | 10 | 176[a] |
|  |  |  | 20 | 20 | 91 |

(continued)

| Metabolite | Retention time (min) | Precursor ion (m/z) | Cone voltage (V) | Collision energy (V) | Product ion (m/z) |
|---|---|---|---|---|---|
| Gluconate | 1.96 | 302 | 20 | 10 | 124[a] |
| | | | 20 | 20 | 91 |
| $\alpha$-ketobutyrate | 5.51 | 313 | 20 | 10 | 190[a] |
| | | | 20 | 20 | 91 |
| Octanoate-$^{13}C_4$ | 5.52 | 254 | 20 | 20 | 124[a] |
| | | | 20 | 10 | 91 |
| $\beta$-hydroxybutyrate-$d_4$ | 3.44 | 214 | 20 | 10 | 124[a] |
| | | | 20 | 20 | 91 |
| Lactate-$d_3$ | 3.26 | 199 | 20 | 5 | 124[a] |
| | | | 20 | 10 | 91 |
| Pyruvate-$^{13}C_3$ | 5.16 | 302 | 20 | 10 | 181[a] |
| | | | 20 | 20 | 91 |

Statistics

The suitability of the selected metabolite signature for discerning between cancer and controls for the different tumors was evaluated by performing a receiver operating characteristic (ROC) and by calculating the corresponding area under the curve (AUC). ROC curves and AUC values were performed using SPSS software (v 18.0; IBM, Armonk, New York, NY, USA).

In order to obtain a population threshold for each selected biomarker, we accurately quantified the concentrations for each metabolite in an extended Control Cohort comprising 134 healthy controls (HC) recruited at Hospital del Mar (Barcelona, Spain) (n=95), Hospital Vall d'Hebrón (Barcelona, Spain) (n = 30) and Hospital Trueta (Girona, Spain) (n=9). This cohort was used to establish both reference and threshold values. Population threshold for each marker of the signature was calculated considering the following equation:

$$\text{Threshold} > (\text{controls concentration average}) + 3 \times (\text{controls standard deviation}).$$

These thresholds would account for at least 99.7% of the population. Thus, individuals with results below the threshold for all metabolites can be considered negative and results above the threshold for any of the biomarkers of the signature can result in a positive case.

Table 7. Concentrations in controls and established threshold for each metabolite

| Biomarker | Controls (n=134) | | Threshold |
|---|---|---|---|
| | Mean | Range* | |
| | ng/mL | | |
| Octanoate | 119 | 2.3-1158 | 2000 |
| Decanoate | 274 | 44 - 1594 | 3000 |
| $\beta$-OH-Butyrate | 7853 | 1997 - 30888 | 50000 |
| Acetoacetate | 2577 | 139 - 11235 | 12500 |
| 3-oxopropanoate | 19 | 6.1 - 67 | 250 |
| Gluconate | 1104 | 42 - 3192 | 5000 |
| $\alpha$-Ketobutyrate | 166 | 20 - 590 | 700 |
| Octanoate + Decanoate | 393 | 82 - 2184 | 4000 |

(continued)

| Biomarker | Controls (n=134) | | Threshold |
| | Mean | Range* | |
| | ng/mL | | |
| β-OH-Butyrate + Acetoacetate | 10430 | 2515 - 40567 | 55000 |
| * Results of range expressed as "minimum - maximum" | | | |

### 1.2. Results

#### 1.2.1. Pancreatic cancer

The MKM metabolic signature containing the biomarkers 3-oxopropanoate, beta-hydroxybutyrate and decanoate was evaluated in a cohort of pancreatic cancer patients (cases, N=80) and non-oncologic patients (controls, which included both healthy subjects and chronic pancreatitis patients, N=36). The MKM signature was able to differentiate cases from controls (AUC =0.962) as can be seen in Figure 1.

The predictive potential of the MKM signature for pancreatic cancer diagnosis was higher than that of CA19.9 (Figure 2A) and by the individual components of the signature (Figures 2B-2D).

In order to check the potential of the MKM signature for the early diagnosis of pancreatic cancer, we selected from the pancreatic cancer cohort those patients diagnosed in stages I and IIA. The early stage cohort of pancreatic cancer consisted on patients (cases, N=26) and non-oncologic patients (controls, which included both healthy subjects and chronic pancreatitis patients, N=36). The MKM signature was able to differentiate cases from controls in a similar way than when using the whole cohort (AUC =0.963) as can be seen in Figure 3.

The potential of the MKM signature for early pancreatic cancer diagnosis was higher than the obtained by CA19.9 (Figure 4A) and by the individual components of the signature (Figures 4B-4D).

An extended MKM metabolic signature was also evaluated. This extended MKM signature contained, in addition to 3-oxopropanoate, beta-hydroxybutyrate and decanoate, the following metabolites: octanoate, acetoacetate, gluconate and alpha-ketobutyrate. Results for the 7 biomarker signature are shown in figures 5-7. It may be seen that the addition of selected biomarkers as disclosed above to the basic MKM signature further improved the discriminating capacity of the diagnostic method of the invention.

The percentage of patients included in the whole pancreatic cancer cohort who had an increased plasma concentration of at least one of the 7 biomarkers was calculated. The same calculation was performed for a group of non-oncologic patients which included both healthy and chronic pancreatitis patients. We also calculated the percentage of patients included in the whole pancreatic cancer cohort who tested positive for plasma CA19.9 and also had increased plasma concentration of at least one of the 7 biomarkers. It was found that 87% of the blood samples from pancreatic cancer patients included in the study had increased levels of at least one of the metabolites (Figure 7B), while only two of the non-oncologic subjects had at least one of the metabolites (6%) (Figure 7A). It was also found that 97% of pancreatic cancer patients included in the study had increased levels of at least one of the metabolites in addition to CA19.9 (Figure 7C).

#### 1.2.2. Gastric cancer

The MKM signature was also evaluated in a cohort of gastric cancer patients (cases, N=127) and non-oncologic patients (controls, N=52). The MKM signature was able to differentiate cases from controls (AUC =0.760) as can be seen in Figure 8A.

The use of the seven markers signature improved the results obtaining an AUC = 0.860 as can be seen in Figure 8B

Similar results were obtained for early diagnosis i.e., when only considering those patients diagnosed in stages 0, I and IIA (N= 48). The MKM signature was able to differentiate cases from controls in a similar way than when using the whole cohort (AUC =0.768) as can be seen in Figure 9A.

The use of the seven markers signature improved the results obtaining an AUC = 0.887 as can be seen in Figure 9B

#### 1.2.3. Esophageal cancer

The MKM signature was also evaluated in a cohort of esophageal cancer patients (cases, N=52) and non-oncologic patients (controls, N=50). The MKM signature was able to differentiate cases from controls (AUC =0.833) as can be seen in Figure 10A.

The use of the seven markers signature improved the results obtaining an AUC = 1.000 as can be seen in Figure 10B

### 1.2.4. Gynecological cancer

The MKM signature was also evaluated in a cohort of gynecological cancer patients (cases, N=9) and non-oncologic patients (controls, N=6). The MKM signature was able to differentiate cases from controls (AUC =1.000) as can be seen in Figure 11A.

The use of the seven markers signature also yielded an AUC = 1.000 as can be seen in Figure 11B

### 1.2.5. Lung cancer

The MKM signature was also evaluated in a cohort of lung cancer patients (cases, N=98) and non-oncologic patients (controls, N=50). The MKM signature was able to differentiate cases from controls (AUC =0.797) as can be seen in Figure 12A.

The use of the seven markers signature improved the results obtaining an AUC = 0.982 as can be seen in Figure 12B

### Citation List

Burtis C. A. et al., 2008, Chapter 14, section "Statistical Treatment of Reference Values"

Gillen S, Schuster T, Büschenfelde CM zum, et al: Preoperative/neoadjuvant therapy in pancreatic cancer: A systematic review and meta-analysis of response and resection percentages. PLoS Medicine 7, 2010. PMID: 20422030 PMCID: PMC2857873.

Gomez-Gomez et al. Determination of up to twenty carboxylic acid containing compounds in clinically relevant matrices by o-benzylhydroxylamine derivatization and liquid chromatography-tandem mass spectrometry. Journal of Pharmaceutical and Biomedical Analysis 2022 Jan 20;208:114450. PMID: 34798391https://doi.org/10.1016/j.jpba.2021.114450

Kenner B, Chari ST, Kelsen D, et al. Artificial Intelligence and Early Detection of Pancreatic Cancer: 2020 Summative Review. Pancreas. 2021;50(3):251-279. PMID: 33835956 PMCID: PMC8041569

Luo G, Jin K, Deng S, Cheng H, Fan Z, Gong Y, Qian Y, Huang Q, Ni Q, Liu C, Yu X. Roles of CA19-9 in pancreatic cancer: Biomarker, predictor and promoter. Biochim Biophys Acta Rev Cancer. 2021 Apr;1875(2):188409. doi: 10.1016/j.bbcan.2020.188409. Epub 2020 Aug 19. PMID: 32827580

Mellby LD, Nyberg AP, Johansen JS, Wingren C, Nordestgaard BG, Bojesen SE, Mitchell BL, Sheppard BC, Sears RC, Borrebaeck CAK. Serum Biomarker Signature-Based Liquid Biopsy for Diagnosis of Early-Stage Pancreatic Cancer. J Clin Oncol. 2018 Oct 1;36(28):2887-2894. doi: 10.1200/JCO.2017.77.6658. Epub 2018 Aug 14. PMID: 30106639; PMCID: PMC6161836.

Park W, Chawla A, O'Reilly EM. Pancreatic Cancer: A Review. JAMA - Journal of the American Medical Association 326:851-862, 2021. PMID: 34547082

Rahib L, Smith BD, Aizenberg R, et al. Projecting cancer incidence and deaths to 2030: The unexpected burden of thyroid, liver, and pancreas cancers in the United States. Cancer Research 74:2913-2921, 2014. PMID: 24840647

Siegel RL, Miller KD, Fuchs HE, Jemal A. Cancer statistics, 2022. CA Cancer J Clin. 2022 Jan;72(1):7-33. doi: 10.3322/caac.21708. Epub 2022 Jan 12. PMID: 35020204.

**Claims**

1. An *in vitro* method for diagnosing cancer in a subject, said method comprising the steps of:

   (i) determining, in a sample obtained from the subject, the concentration of (a) 3-oxopropanoate, and at least one further metabolite selected from (b) a ketone body selected from beta-hydroxybutyrate, acetoacetate, and combinations thereof, and (c) a medium chain fatty acid selected from octanoate, decanoate, and combinations thereof,
   (ii) comparing the concentration of each of the metabolites determined in (i) with a corresponding reference value, and
   (iii) diagnosing the patient as having cancer when the concentration of at least one of the metabolites is increased as compared to the corresponding reference value.

2. The *in vitro* method according to claim 1, wherein step (i) comprises determining (a), (b) and (c).

3. The *in vitro* method according to any one of claims 1-2, wherein step (i) comprises determining 3-oxopropanoate, beta-hydroxybutyrate, acetoacetate, octanoate, and decanoate.

4. The *in vitro* method according to any one of claims 1-3, wherein at least (a) is increased as compared to the corresponding reference value.

5. The *in vitro* method according to any one of claims 1-4, wherein step (i) further comprises determining the concentration of a metabolite selected from (d) alpha-ketobutyrate, (e) gluconate, and combinations thereof.

6. The *in vitro* method according to any one of claims 1-5, wherein the increase is at least 3-fold, in particular at least 4-fold.

7. The *in vitro* method according to any one of claims 1-6, wherein the reference value for each metabolite is the concentration of said metabolite determined in the biological sample obtained from a non-oncologic subject or group of subjects.

8. The *in vitro* method according to any one of the preceding claims, wherein the subject is diagnosed as having early stage cancer, in particular, cancer in stages IA, IB or IIA.

9. The *in vitro* method according to any one of the preceding claims, wherein the method is **characterized by** a receiver operating characteristic area under the curve value in the range from 0.800 to 1.000, in particular in the range from 0.900 to 1.000.

10. The *in vitro* diagnosis according to any one of the preceding claims, that is for diagnosing a solid tumor.

11. The *in vitro* diagnosis according to the preceding claim, wherein the cancer is selected from the group consisting of pancreatic cancer, gastric cancer, esophageal cancer, gynecological cancer, breast cancer, colorectal cancer, and lung cancer, in particular pancreatic cancer, more particularly pancreatic ductal adenocarcinoma.

12. The *in vitro* diagnosis according to any one of the preceding claims, wherein the sample is selected from blood, plasma and serum.

13. The *in vitro* diagnosis according to any one of the preceding claims, wherein the amount of metabolite is determined by liquid chromatography with tandem mass spectrometry.

14. A kit of parts for diagnosing cancer as defined in any one of claims 1-13, said kit comprising:

    (i) means for determining the concentration of (a) 3-oxopropanoate, and at least one other metabolite selected from (b) a ketone body selected from beta-hydroxybutyrate, acetoacetate and combinations thereof, and (c) a medium chain fatty acid selected from octanoate, decanoate and combinations thereof, and optionally means for determining the concentration of a further metabolite selected from (d) alpha-ketobutyrate, (e) gluconate, and combinations thereof,
    (ii) optionally, reference values for the above metabolites, and

(iii) instructions for diagnosing cancer.

15. Combined use of (a) 3-oxopropanoate and at least one of (b) a ketone body selected from beta-hydroxybutyrate, acetoacetate and combinations thereof, or (c) a medium chain fatty acid selected from octanoate, decanoate and combinations thereof, as a biomarker for the diagnosis of cancer.

**MKM Signature**

AUC: 0.962
95% CI: 0.929 – 0.995

Figure 1

Figure 2

**MKM Signature**

AUC: 0.963
95% CI: 0.923 − 1.000

Figure 3

**(A)**

CA 19.9

AUC: 0.646
95% CI: 0.469 – 0.823

**(B)**

MCFA (Decanoic acid)

AUC: 0.675
95% CI: 0.531 – 0.819

**(C)**

3-oxopropanoate

AUC: 0.851
95% CI: 0.757 – 0.946

**(D)**

KB (βOH-Butyrate)

AUC: 0.843
95% CI: 0.749 – 0.937

Figure 4

7-biomarker signature

AUC: 0.981
95% CI: 0.956 − 1.000

Figure 5

**(A)**

**Controls vs PDAC early stages**

AUC: 1.000
95% CI: 1.000 − 1.000

**(B)**

**ChrP vs PDAC early stages**

AUC: 0.978
95% CI: 0.948 − 1.000

Figure 6

(A)

(B)

6%

94%

13%

87%

(C)

(D)

3%

97%

45%

55%

(E)

(F)

25%

75%

39%

61%

Figure 7

Figure 8

Figure 9

Figure 10

Figure 11

**(A)**

**MKM Signature**

AUC: 0.797
95% CI: 0.726 – 0.867

**(B)**

**7 biomarker signature**

AUC: 0.982
95% CI: 0.965 – 0.999

Figure 12

EP 4 246 146 A1

Europäisches
Patentamt

European
Patent Office

Office européen
des brevets

**EUROPEAN SEARCH REPORT**

Application Number

EP 22 38 2244

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Akhbari ET AL: "Can joint fluid metabolic profiling (or "metabonomics") reveal biomarkers for osteoarthritis and inflammatory joint disease?", Bone Joint Res. 2020;9(3), 3 March 2020 (2020-03-03), pages 108-119, XP055951656, DOI: 10.1302/2046-3758.93 Retrieved from the Internet: URL:https://www.google.com/url?sa=t&rct=j& q=&esrc=s&source=web&cd=&ved=2ahUKEwjh_KTp 3Mr5AhU0XfEDHW-bD0U4ChAWegQIGRAB&url=https %3A%2F%2Fonline.boneandjoint.org.uk%2Fdoi% 2Fpdf%2F10.1302%2F2046-3758.93.BJR-2019-01 67.R1%3Fdownload%3Dtrue&usg=AOvVaw28KzFvxJ IpJME4RjClMCO2 [retrieved on 2022-08-16] * figure 2b * | 1-15 | INV. G01N33/574 |
| X | CN 107 064 508 A (UNIV SHENZHEN) 18 August 2017 (2017-08-18) * paragraphs [0142] - [0149]; claims 1-2,7 * | 1-15 | TECHNICAL FIELDS SEARCHED (IPC) G01N |
| X | WO 2016/051020 A1 (BIOSCIENCES OY) 7 April 2016 (2016-04-07) * page 29, lines 3-8; claims 1-3,6; figures 1,5,7 * | 1-15 | |
| X | SHIN NISHIUMI ET AL: "Metabolomics for Biomarker Discovery in Gastroenterological Cancer", METABOLITES, vol. 4, no. 3, 7 July 2014 (2014-07-07), pages 547-571, XP055411796, DOI: 10.3390/metabo4030547 * Table 1 on page 552, 553, 554,558 * | 1-15 | |

-/--

The present search report has been drawn up for all claims

| Place of search | Date of completion of the search | Examiner |
|---|---|---|
| The Hague | 16 August 2022 | van der Kooij, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

31

## EUROPEAN SEARCH REPORT

**Application Number**

**EP 22 38 2244**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/059571 A1 (LAM CHING-WAN [HK]) 2 March 2017 (2017-03-02) * paragraphs [0007], [0023], [0036]; claims 1,9; figure 4; example 1 * ----- | 1-15 | |
| X | IOLE MARIA DI GANGI ET AL: "Analytical metabolomics-based approaches to pancreatic cancer", TRAC TRENDS IN ANALYTICAL CHEMISTRY, vol. 55, 1 March 2014 (2014-03-01), pages 94-116, XP055715364, AMSTERDAM, NL ISSN: 0165-9936, DOI: 10.1016/j.trac.2013.12.006 * table 2, pages 105,106 * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 16 August 2022 | van der Kooij, M |

## ANNEX TO THE EUROPEAN SEARCH REPORT
## ON EUROPEAN PATENT APPLICATION NO.

EP 22 38 2244

This annex lists the patent family members relating to the patent documents cited in the above-mentioned European search report.
The members are as contained in the European Patent Office EDP file on
The European Patent Office is in no way liable for these particulars which are merely given for the purpose of information.

16-08-2022

| Patent document cited in search report | | Publication date | Patent family member(s) | | Publication date |
|---|---|---|---|---|---|
| CN 107064508 | A | 18-08-2017 | NONE | | |
| WO 2016051020 | A1 | 07-04-2016 | AU | 2015326756 A1 | 11-05-2017 |
| | | | BR | 112017006593 A2 | 19-12-2017 |
| | | | CA | 2959832 A1 | 07-04-2016 |
| | | | CN | 106716127 A | 24-05-2017 |
| | | | EP | 3201623 A1 | 09-08-2017 |
| | | | JP | 6892820 B2 | 23-06-2021 |
| | | | JP | 2017535756 A | 30-11-2017 |
| | | | KR | 20170061704 A | 05-06-2017 |
| | | | SG | 11201701875Y A | 27-04-2017 |
| | | | US | 2017285036 A1 | 05-10-2017 |
| | | | WO | 2016051020 A1 | 07-04-2016 |
| US 2017059571 | A1 | 02-03-2017 | EP | 3136100 A2 | 01-03-2017 |
| | | | EP | 3444614 A1 | 20-02-2019 |
| | | | US | 2017059571 A1 | 02-03-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SIEGEL RL et al.** *CA Cancer J Clin.,* January 2022, vol. 72 (1), 7-33
- **RAHIB L et al.** *Cancer Research,* June 2014, vol. 74 (11), 2913-2921
- **PARK W et al.** *JAMA - Journal of the American Medical Association,* September 2021, vol. 326 (9), 851-862
- **KENNER B et al.** *Pancreas,* March 2021, vol. 50 (3), 251-279
- **GILLEN S et al.** *PLoS Medicine,* April 2010, vol. 7 (4), e1000267
- **LUO G et al.** *Biochim Biophys Acta Rev Cancer,* April 2021, vol. 1875 (2), 188409
- **MELLBY et al.** *J Clin Oncol,* 01 October 2018, vol. 36 (28), 2887-2894
- **BURTIS C. A. et al.** Statistical Treatment of Reference Values. 2008
- **GÓMEZ-GÓMEZ et al.** *Journal of Pharmaceutical and Biomedical Analysis,* January 2022, vol. 208, 114450
- **GILLEN S ; SCHUSTER T ; BÜSCHENFELDE CM ZUM et al.** Preoperative/neoadjuvant therapy in pancreatic cancer: A systematic review and meta-analysis of response and resection percentages. *PLoS Medicine,* 2010, vol. 7
- **GOMEZ-GOMEZ et al.** Determination of up to twenty carboxylic acid containing compounds in clinically relevant matrices by o-benzylhydroxylamine derivatization and liquid chromatography-tandem mass spectrometry. *Journal of Pharmaceutical and Biomedical Analysis,* 20 January 2022, vol. 208, 114450, https://doi.org/10.1016/j.jpba.2021.114450
- **KENNER B ; CHARI ST ; KELSEN D et al.** Artificial Intelligence and Early Detection of Pancreatic Cancer: 2020 Summative Review. *Pancreas,* 2021, vol. 50 (3), 251-279
- **LUO G ; JIN K ; DENG S ; CHENG H ; FAN Z ; GONG Y ; QIAN Y ; HUANG Q ; NI Q ; LIU C.** Roles of CA19-9 in pancreatic cancer: Biomarker, predictor and promoter. *Biochim Biophys Acta Rev Cancer,* 19 August 2020, vol. 1875 (2), 188409
- **MELLBY LD ; NYBERG AP ; JOHANSEN JS ; WINGREN C ; NORDESTGAARD BG ; BOJESEN SE ; MITCHELL BL ; SHEPPARD BC ; SEARS RC ; BORREBAECK CAK.** Serum Biomarker Signature-Based Liquid Biopsy for Diagnosis of Early-Stage Pancreatic Cancer. *J Clin Oncol.,* 14 August 2018, vol. 36 (28), 2887-2894
- **PARK W ; CHAWLA A ; O'REILLY EM.** Pancreatic Cancer: A Review. *JAMA - Journal of the American Medical Association,* 2021, vol. 326, 851-862
- **RAHIB L ; SMITH BD ; AIZENBERG R et al.** Projecting cancer incidence and deaths to 2030: The unexpected burden of thyroid, liver, and pancreas cancers in the United States. *Cancer Research,* 2014, vol. 74, 2913-2921
- **SIEGEL RL ; MILLER KD ; FUCHS HE ; JEMAL A.** *CA Cancer J Clin.,* 12 January 2022, vol. 72 (1), 7-33